# EUROPEAN PATENT APPLICATION

(11) **EP 3 348 667 A1**
(43) Date of publication of application: **18.07.2018**
(21) Application number: 16844481.8
(22) Date of filing: 09.09.2016
(51) Int. Cl.: C23C 16/18, C07C 251/08, C07F 15/00, H01L 21/285

(54) **CHEMICAL VAPOR DEPOSITION FEEDSTOCK COMPRISING ORGANIC RUTHENIUM COMPOUND AND CHEMICAL VAPOR DEPOSITION METHOD USING SAID CHEMICAL VAPOR DEPOSITION FEEDSTOCK**

(30) Priority: 11.09.2015 JP 2015180101
(71) Applicant: Tanaka Kikinzoku Kogyo K.K., Chiyoda-ku Tokyo 100-6422 (JP)
(72) Inventor: HARADA, Ryosuke, Tsukuba-shi Ibaraki 300-4247 (JP); SHIGETOMI, Toshiyuki, Tsukuba-shi Ibaraki 300-4247 (JP); ISHIZAKA, Tasuku, Tsukuba-shi Ibaraki 300-4247 (JP); AOYAMA, Tatsutaka, Tsukuba-shi Ibaraki 300-4247 (JP)
(74) Representative: Weickmann & Weickmann PartmbB
(86) International application number: PCT/JP2016/076585
(87) International publication number: WO 2017/043620

(57) **Abstract**

The present invention relates to a chemical vapor deposition raw material for producing a ruthenium thin film or a ruthenium compound thin film by a chemical deposition method, including an organoruthenium compound in which two diazadiene ligands and two alkyl ligands are coordinated to ruthenium, the organoruthenium being represented by the following formula. The organoruthenium compound to be applied in the present invention can be used for low-temperature deposition, and capable of forming a ruthenium thin film or a ruthenium compound thin film without use of an oxygen gas.

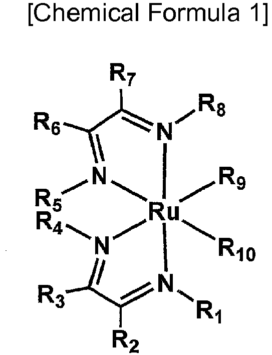

## Description

### Technical Field

The present invention relates to a chemical vapor deposition raw material including an organoruthenium compound, which is used for producing a ruthenium thin film or a ruthenium compound thin film by a chemical deposition method (chemical vapor deposition method (CVD method) or an atomic layer deposition method (ALD method)). Specifically, the present invention relates to a chemical vapor deposition raw material having a low decomposition temperature and moderate thermal stability.

### Background Art

As a thin film electrode material of a semiconductor device such as DRAM or FERAM, ruthenium or a ruthenium compound is used. As a method for producing such a thin film, a chemical deposition method such as a CVD method (chemical vapor deposition method) or an ALD method (atomic layer deposition method) is applied. As raw material compounds that are used in such a chemical deposition method, many compounds including an organoruthenium compound have been heretofore known.

As an organoruthenium compound as a chemical vapor deposition raw material, for example, Patent Document 1 discloses bis(ethylcyclopentadienyl)ruthenium (II) of Chemical Formula 1 in which a cyclopentadienyl group being cyclic dienyl is coordinated. Patent Document 2 discloses bis(2,4-dimethylpentadienyl)ruthenium (II) of Chemical Formula 2 in which a pentadienyl group being chain dienyl is coordinated.

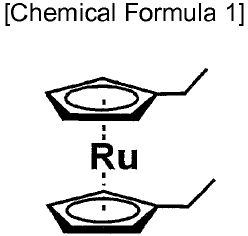

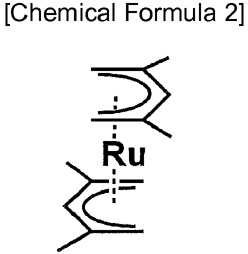

In addition, Patent Document 3 discloses benzene(glyoxal-bis-isopropylamine)ruthenium (II) of Chemical Formula 3 in which a benzene ring and a diazadiene group are coordinated to ruthenium.

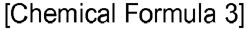

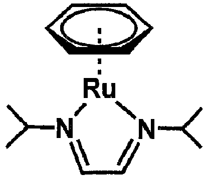

Further, as a ligand that is coordinated to ruthenium, mention is also made of an organoruthenium compound in which a β-diketonato ligand containing an oxygen atom in its structure. For example, Patent Document 4 discloses carbonyl-bis(tetramethylheptanedionato)ruthenium of Chemical Formula 4 in which tetramethylheptanedionato and carbonyl are coordinated. In addition, mention is also made of tris(acetylacetonato)ruthenium of Chemical Formula 5 in which three acetylacetonatos are coordinated as a β-diketonato ligand.

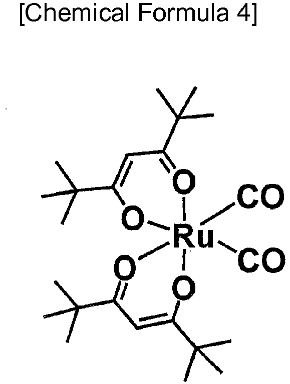

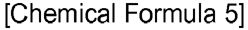

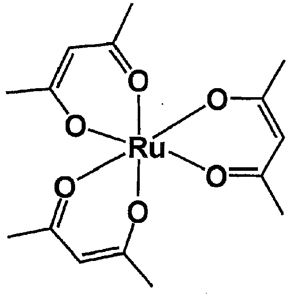

### Related Art Document

### Patent Documents

Patent Document 1: JP 2000-281694 A
Patent Document 2: JP 2010-215982 A
Patent Document 3: WO 2013/117955 A1
Patent Document 4: US 6303809 B1

### Summary of the Invention

### Problems to be Solved by the Invention

Main properties required for organoruthenium compounds for chemical deposition used to be fundamental properties such as possibility and efficiency in formation of a ruthenium thin film, and handling property. In a chemical deposition method, a raw material compound is vaporized into a raw material gas, and the raw material gas is transported to a substrate. A compound having a high vapor pressure is considered suitable for efficiently forming a thin film. In addition, with consideration given to the handling property of the raw material, a compound which has high thermal stability and is not easily decomposed is considered preferable.

However, there is no end to requirements for miniaturization and performance improvement of semiconductor devices, and those that are more advanced before are constantly being developed. For keeping up with this trend, characteristics demanded for organoruthenium compounds as chemical vapor deposition raw materials have been changed. As such novel demand characteristics, mention is made of problems with a requirement for decreasing a deposition temperature and selection of a reaction gas. All of these problems are related to damage to a substrate in formation of a ruthenium thin film, and quality of a thin film.

The organoruthenium compound of Chemical Formula 1 has a high vapor pressure and high thermal stability, and is therefore a suitable organoruthenium compound from a traditional viewpoint. Further, the organoruthenium compound is liquid at normal temperature, which also contributes to high usefulness of the organoruthenium compound. However, since the organoruthenium compound has a high decomposition temperature of 350°C, the deposition temperature cannot be set low.

In addition, the organoruthenium compound has the problem that ruthenium cannot be precipitated unless oxygen is introduced as a reaction gas. Use of an oxygen gas may cause oxidation of a substrate composed of silicon or the like, so that concerns remain that device properties may be affected.

The organoruthenium compound of Chemical Formula 2 is also a suitable compound from the viewpoint of a vapor pressure although it is solid at normal temperature. However, the organoruthenium compound has a problem in practical use because it has poor thermal stability, and is easily decomposed when heated at about 80°C. Further, the organoruthenium compound also requires oxygen as a reaction gas.

In contrast to the above-mentioned organoruthenium compounds, the organoruthenium compound of Chemical Formula 3 has no problem with a reaction gas because a ruthenium thin film can be formed with hydrogen as a reaction gas. However, this compound has a disadvantage that it has a low vapor pressure, leading to poor basic properties as a chemical vapor deposition raw material. The vapor pressure level is property related to deposition efficiency, and is a condition prerequisite from the viewpoint of industrial utilization of a chemical deposition method.

The organoruthenium compounds of Chemical Formulae 4 and 5 are highly appreciated in that the hydrogen can be applied as a reaction gas, but it is apprehended that the structure of the ligand contains an oxygen atom. In the case of the organoruthenium compounds containing an oxygen atom, there is not a problem in the effect of the substrate by the reaction gas, but ingress of the oxygen atom of the ligand into a ruthenium thin film may occur. Oxygen in the ruthenium thin film is considered to affect electrode properties. In Patent Document 4, ingress of oxygen in the thin film is mentioned, and it is shown that a ruthenium thin film produced from the compound of Chemical Formula 4 contains oxygen in an amount of about 3%. The organoruthenium compound of Chemical Formula 4 is also a compound which can hardly be used for low-temperature deposition, so that it is required to set a considerably high deposition temperature of 400°C or higher.

As described above, organoruthenium compounds which have been heretofore applicable as chemical vapor deposition raw materials may have both advantages and disadvantages with respect to diversified required properties. Thus, the present invention provides an organoruthenium compound which is usable for low-temperature deposition while having basic properties as a chemical vapor deposition raw material. The present invention also provides an organoruthenium compound which is capable of forming a ruthenium thin film without use of an oxygen gas, so that a substrate and the resulting ruthenium thin film are hardly affected. As a specific guideline for low-temperature deposition in the present invention, deposition can be performed at 250°C or lower.

### Means for Solving the Problems

The present invention for solving the above-described problems provides a chemical vapor deposition raw material for producing a ruthenium thin film or a ruthenium compound thin film by a chemical deposition method, including an organoruthenium compound in which two diazadiene ligands and two alkyl ligands are coordinated to ruthenium, the organoruthenium being represented by the following formula: 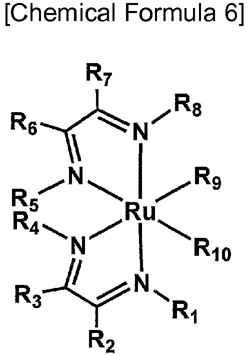 in which each of substituents R₁ to R₈ of the diazadiene ligand is hydrogen, or a hydrocarbon group with a carbon number of 1 or more and 4 or less; two or more substituents selected from substituents R₁ to R₄ may be bonded together to form a cyclic structure with a carbon atom or a nitrogen atom to which the substituents are directly bonded; two or more substituents selected from substituents R₅ to R₈ may be bonded together to form a cyclic structure with a carbon atom or a nitrogen atom to which the substituents are directly bonded; and each of substituents R₉ and R₁₀ as alkyl ligands is an alkyl group with a carbon number of 1 or more and 3 or less.

The chemical vapor deposition raw material of the present invention includes an organoruthenium compound having both a diazadiene ligand and an alkyl ligand. Application of these ligands is intended for making it possible to perform low-temperature deposition under a hydrogen atmosphere with thermal stability set in an appropriate range in consideration of the strength of the bond of each ligand to ruthenium. That is, a diazadiene-ruthenium bond with a strong bonding force and an alkyl-ruthenium bond with a relatively weak bonding force are introduced into the structure of the organoruthenium compound to control the physical properties of the compound as a whole.

The organoruthenium compound includes a diazadiene ligand composed of nitrogen, carbon and hydrogen, an alkyl ligand composed of carbon and hydrogen, and ruthenium, and does not contain an oxygen atom. Therefore, the resulting ruthenium thin film does not contain oxygen derived from a raw material, and will not oxidize a substrate.

The organoruthenium compound which is applied in the present invention has an appropriately high vapor pressure that is basic property required as a chemical vapor deposition raw material. This is ascribable to the fact that each of substituents R₉ and R₁₀ as alkyl ligands is limited to a relatively short-chain alkyl group with a carbon number of 1 or more and 3 or less while each of substituents R₁ to R₈ in the diazadiene ligand is a hydrocarbon group with a carbon number of 1 or more and 4 or less.

The configurations of the chemical vapor deposition raw materials according to the present invention, which have the above-mentioned advantages, will be described in detail below.

In the organoruthenium compound that is applied in the present invention, two diazadiene ligands are coordinated. Each of substituents R₁ to R₈ in the diazadiene ligand is hydrogen, or a hydrocarbon group with a carbon number of 1 or more and 4 or less. All of substituents R₁ to R₈ may be hydrogen, and at least any one of substituents R₁ to R₈ may be a hydrocarbon group. The hydrocarbon group is a substituent composed of hydrogen and carbon.

The reason why each of substituents R₁ to R₈ in the diazadiene ligand is limited to one with a carbon number of 1 or more and 4 or less is that the carbon chains of the substituents R₁ to R₈ may affect the vapor pressure of the organoruthenium compound. When the carbon number is excessively large, the vapor pressure may decrease.

When each of substituents R₁ to R₈ is a hydrocarbon group, examples of the substituents include linear or branched alkyl groups, vinyl groups and allyl groups. When each of substituents R₁ to R₈ is a hydrocarbon group, these substituents may be independent substituents, or may be bonded together. That is, two or more substituents selected from substituents R₁ to R₄ may be bonded together to form a cyclic structure with a carbon atom or a nitrogen atom to which the substituents are directly bonded. Similarly, two or more substituents selected from substituents R₅ to R₈ may be bonded together to form a cyclic structure with a carbon atom or a nitrogen atom to which the substituents are directly bonded. For example, the scope of the present invention also includes an organoruthenium compound in which a diazadiene ligand having a cyclic substituent is coordinated as shown below.

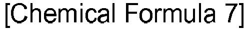

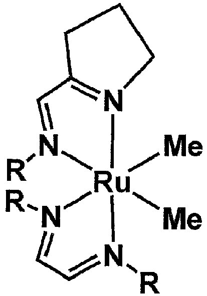

A more preferable configuration of the organoruthenium compound according to the present invention is one in which at least any of substituents R₁, R₄, R₅ and R₈ is an alkyl group, and at least any of the alkyl groups is a linear or branched alkyl group with a carbon number of 1 or more and 4 or less. All of substituents R₁, R₄, R₅ and R₈ may be alkyl groups with a carbon number of 1 or more and 4 or less, or some of substituents R₁, R₄, R₅ and R₈ may be alkyl groups with a carbon number of 1 or more and 4 or less, with the other substituents being hydrogen or other alkyl groups. Examples of the linear or branched alkyl group include a methyl group, an ethyl group, a n-propyl group, an iso-propyl group, a n-butyl group, an iso-butyl group, a sec-butyl group and a tert-butyl group.

Further, when at least any of substituents R₂, R₃, R₆ and R₇ is an alkyl group, at least any of the alkyl groups is preferably a methyl group. All of substituents R₂, R₃, R₆ and R₇ may be methyl groups, or some of substituents R₂, R₃, R₆ and R₇ may be methyl groups, with the other substituents being hydrogen or other alkyl groups.

In the organoruthenium compound according to the present invention, each of alkyl ligands R₉ and R₁₀ as other ligands is an alkyl group with a carbon number of 1 or more and 3 or less (methyl group, ethyl group, n-propyl group or iso-propyl group) The length of the carbon chain of the alkyl ligand affects the level of the vapor pressure of the organoruthenium compound. By limiting the ligand to a short-chain alkyl group, a suitable vapor pressure can be secured.

A chemical deposition method of a ruthenium thin film or a ruthenium compound thin film in which the chemical deposition raw material according to the present invention is applied will now be described. In the present chemical deposition method, a raw material including an organoruthenium compound as described above is vaporized by heating to generate a raw material gas, the raw material gas is transported onto a substrate surface, and the organoruthenium compound is thermally decomposed to form a ruthenium thin film.

With regard to the form of the raw material in the chemical deposition method, the organoruthenium compound that is applied in the present invention may be solid at normal temperature, but has a high vapor pressure, and thus can be easily vaporized by a sublimation method. Therefore, the organoruthenium compound as a raw material can be heated as it is. In addition, a raw material gas can also be obtained by solving the raw material in an appropriate solvent, and heating the solution. The raw material heating temperature is preferably 50°C or higher and 150°C or lower.

Normally, the vaporized raw material joins with a carrier gas, and is transported onto the substrate. The organoruthenium compound according to the present invention enables ruthenium to be deposited with an inert gas (argon, nitrogen or the like) as a carrier gas even when a reaction gas is not used.

In addition, a reaction gas can be set as necessary. In production of a thin film including ruthenium, the chemical vapor deposition raw material according to the present invention can be deposited even when oxygen is not used. Here, a reducing gas species such as hydrogen, ammonia, hydrazine or formic acid can be applied as a reaction gas. However, application of oxygen as a reaction gas is not excluded. In deposition of a ruthenium compound thin film of ruthenium oxide or the like, an oxygen gas can be applied as a reaction gas. Such a reaction gas can also serve as a carrier gas.

The deposition temperature during deposition is preferably 150°C or higher and 500°C or lower. When the deposition temperature is lower than 150°C, a deposition reaction hardly proceeds, so that efficient deposition cannot be performed. In contrast, when the deposition temperature is excessively high, there is the problem that uniform deposition is difficult, the substrate may be damaged, or the like. Normally, the deposition temperature is adjusted by the substrate heating temperature. Of course, when consideration is given to attainment of low-temperature decomposition which is an object of the present invention, the decomposition temperature is more preferably 150°C or higher and 400°C or lower, still more preferably 150°C or higher and 300°C or lower.

### Advantageous Effects of the Invention

As described above, the organoruthenium compound that forms the chemical vapor deposition raw material according to the present invention has thermal stability with a degree required for the chemical vapor deposition raw material thanks to selection of a ligand to be coordinated to ruthenium. For the thermal stability, the organoruthenium compound according to the present invention has stability midway between the stability of the compound of Chemical Formula 2 and the stability of the compound of Chemical Formula 3 (Chemical Formula 3 > compound of the present application > Chemical Formula 2). Thus, the organoruthenium compound has moderate stability for handling the compound as a chemical vapor deposition raw material. In addition, the organoruthenium compound has a suitable vapor pressure as a result of adjusting ligands and substituents thereof.

Further, the chemical vapor deposition raw material according to the present invention enables a ruthenium thin film to be deposited by applying a reaction gas other than oxygen, e.g. applying hydrogen. In addition, the constituent elements do not include an oxygen atom. Therefore, ingress of oxygen atoms into the produced ruthenium thin film does not occur, and there is no concern of oxidation damage to the substrate.

Thus, the chemical vapor deposition raw material according to the present invention is useful for formation of highly miniaturized semiconductor devices of recent years.

### Brief Description of the Drawings

Fig. 1 illustrates results of TG-DTA measurement of a compound in a first embodiment (under reduced pressure).
Fig. 2 illustrates results of TG-DTA measurement of a compound in the first embodiment (under atmospheric pressure only in TG).
Fig. 3 shows a SEM photograph of a ruthenium thin film (gas: hydrogen) deposited at a deposition temperature of 250°C.
Fig. 4 shows a SEM photograph of a ruthenium thin film (gas: argon) deposited at a deposition temperature of 250°C.
Fig. 5 illustrates a result of TG-DTA measurement of a compound in the second embodiment (under atmospheric pressure only in TG).

### Description of Embodiment

First embodiment: Hereinafter, the best embodiments in the present invention will be described. In this embodiment, cis-dimethyl-bis(N,N'-diisopropyl-1,4-diaza-1,3-butadiene)ruthenium (II) (each of substituents R₁, R₄, R₅ and R₈ is an isopropyl group, and each of substituents R₉ and R₁₀ is a methyl group) was produced as an organoruthenium compound.

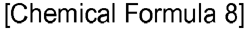

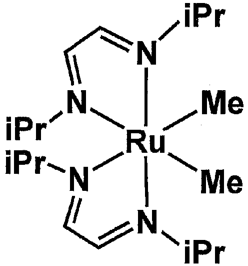

3.80 g (8.4 mmol) of cis-dichloro-bis(N,N'-diisopropyl-1,4-diaza-1,3-butadiene)ruthenium (II) was added in a flask containing 150 mL of tetrahydrofuran, and dissolved, and the solution was cooled with ice. 33 mL of a methylmagnesium iodide/diethyl ether solution (35 mmol; concentration: 1.06 M) was added to the ice-cooled solution. The mixed solution was stirred at room temperature for 1 hour to be reacted. The reaction formula here is as described below.

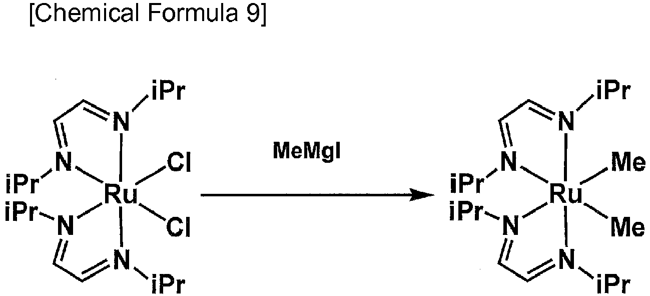

After completion of the reaction, the reaction product was concentrated to obtain a muddy reaction mixture. The resulting muddy reaction mixture was subjected to extraction operation with hexane, and the extract liquid was concentrated to obtain a reddish-violet solid. The solid was purified by a sublimation method to obtain 2.25 g (5.5 mmol) of an organoruthenium compound as a specified substance to be produced (yield: 65%). The organoruthenium compound was definitely confirmed to be cis-dimethyl-bis(N,N'-diisopropyl-1,4-diaza-1,3-butadiene)ruthenium (II) from ¹H-NMR analysis (¹H NMR(benzene-d₆): 8.11(2H, s), 7.80(2H, s), 5.13-5.07(2H, m), 3.79-3.73(2H, m), 1.39(6H, d), 1.36(6H, d), 1.11 (6H, d), 0.65(6H, d), 0.57(6H, s)).

For the organoruthenium compound produced as described above, physical properties were evaluated, and a deposition test was conducted.

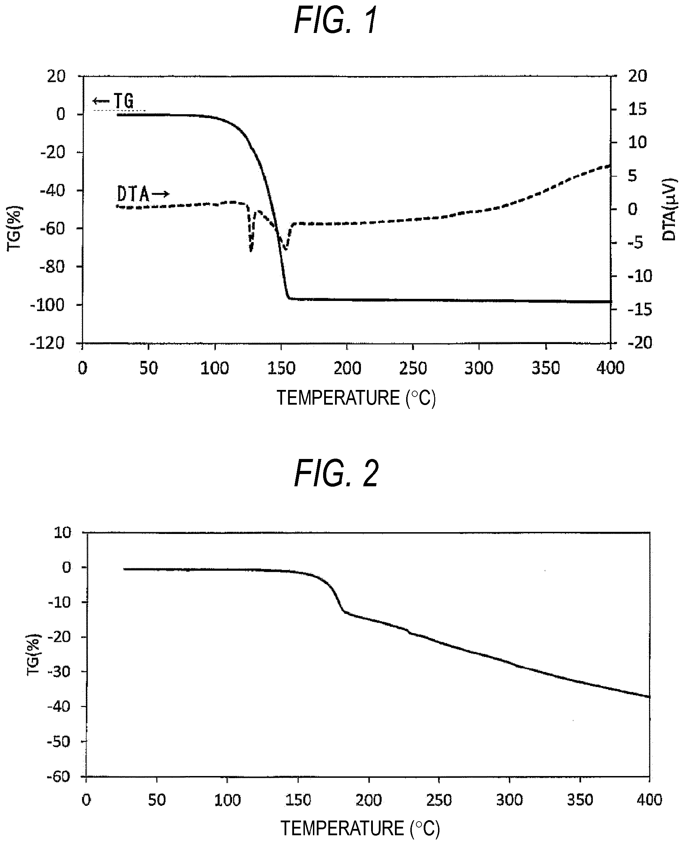

Differential thermal analysis-thermogravimetry: In TG-DTA2000SA manufactured by BRUKER Corporation, the organoruthenium compound (sample weight: 5 mg) was packed in an aluminum cell, and a change in weight was observed over a measurement temperature range, i.e. from room temperature to 500°C, at a temperature elevation rate of 5°C/min. The TG-DTA measurement was performed under a reduced pressure (pressure: 5 torr). The results are shown in Fig. 1.

The TG measurement result in Fig. 1 shows that vaporization of cis-dimethyl-bis(N,N'-diisopropyl-1,4-diaza-1,3-butadiene)ruthenium (II) as the organoruthenium compound produced in this embodiment starts at a temperature just above 110°C and ends at a temperature below 200°C. In addition, from the DTA measurement result, an endothermic peak at a temperature of about 150°C may result from vaporization, and rapid vaporization may occur at this temperature. It was confirmed that after the TG-DTA analysis, residues did not remain on the cell, and the organoruthenium compound had favorable vaporization property as a chemical vapor deposition raw material.

The TG-DTA analysis was also performed under an atmospheric pressure condition in accordance with the same method as described above, and consequently, a result as shown in Fig. 2 (only a result of TG) was obtained. The organoruthenium compound produced in this embodiment was confirmed to be decomposed at a relatively low temperature even under an atmospheric pressure.

Decomposition test: A ruthenium thin film was formed by a CVD apparatus (hot wall-type CVD deposition apparatus) by use of the organoruthenium compound according to the present invention as a raw material. Deposition conditions are as described below.

Substrate: Ta/TH-Ox/Si (thermally oxidized Si + Ta)
Deposition temperature: 250°C and 300°C
Sample temperature (vaporization temperature): 95°C
Pressure: 5 Torr
Gas: hydrogen or argon gas
Gas flow rate: 20 sccm
Deposition time: 60 min

As described above, a ruthenium thin film was deposited while the deposition temperature and the reaction gas were changed, and the thickness and the oxygen concentration in the thin film were measured. The thickness of the ruthenium thin film was determined by measuring thicknesses at a plurality of spots in a result of observation with a SEM (scanning electron microscope), and calculating an average of the measured thicknesses. In addition, the oxygen concentration was measured by SIMS (secondary-ion mass spectrometry: ADEPT-1010 manufactured by ULVAC-PHI, Inc.). The results are shown in Table 1.

**[Table 1]**

| Test No. | Deposition temperature | Reaction gas | Thickness/nm | Oxygen concentration/ppm |
|---|---|---|---|---|
| 1 | 250°C | Hydrogen | 8.9 | ND |
| 2 | | Argon | 9.9 | ND |
| 3 | 300°C | Hydrogen | 17.9 | ND |
| 4 | | Argon | 40.7 | ND |

| | | | | |
|---|---|---|---|---|
| ND: Not Detected | | | | |

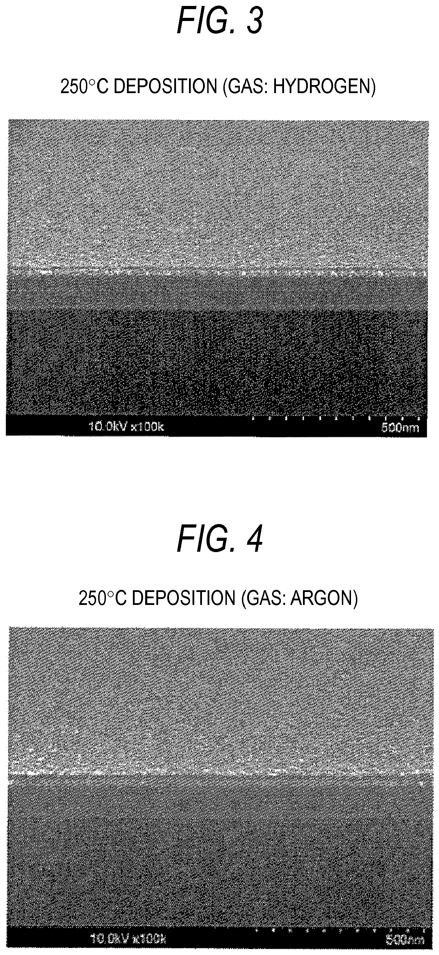

From the results of tests No. 1 and No. 2, it was confirmed that a ruthenium thin film was deposited even when the deposition temperature was set to a low temperature of 250°C. In addition, it is apparent that a ruthenium thin film can be deposited even when hydrogen is used as a reaction gas, and can be deposited even with argon. As in tests No. 3 and No. 4, oxygen was not detected in the ruthenium thin film even when the ruthenium thin film was deposited at a relatively high temperature of 300°C. Figs. 3 and 4 each show a SEM photograph of a ruthenium thin film deposited at a deposition temperature of 250°C.

The specific resistance was measured for each of the thin films obtained by low-temperature deposition in tests No. 1 and No. 2, and the result showed that the specific resistance was 50 µΩ•cm, and thus satisfactory electrical property was exhibited.

Comparative example: As a conventional art, a ruthenium thin film was produced with the tris(acetylacetonato)ruthenium of Chemical Formula 5 as a raw material. Conditions were set as described below. In the comparative example, deposition was also performed at 400°C and 500°C because it was predicted that deposition would not be achieved at 300°C or lower. The results of the deposition test are shown in Table 2.

Substrate: Ta/TH-Ox/Si (thermally oxidized Si)
Deposition temperature: 250°C, 300°C, 400°C and 500°C
Sample temperature (vaporization temperature): 140°C
Pressure: 5 Torr
Reaction gas: hydrogen
Reaction gas flow rate: 20 sccm
Deposition time: 15 min

**[Table 2]**

| Test No. | Deposition temperature | Reaction gas | Ru thickness/nm | Oxygen concentration/% |
|---|---|---|---|---|
| 5 | 250°C | Hydrogen | - | - |
| 6 | 300°C | | - | - |
| 7 | 400°C | | 12.2 | 10 |
| 8 | 500°C | | 11.7 | 10 |

| | | | | |
|---|---|---|---|---|
| -: Unmeasurable because deposition was not achieved. | | | | |

In the case of the organoruthenium compound of the comparative example, when the deposition temperature was low (250°C), ruthenium was not precipitated, and thus it was unable to obtain a thin film (test No. 5). Further, even when the deposition temperature was increased to 300°C, it was unable to deposit ruthenium (test No. 6). These results showed that it was unable to perform low-temperature deposition with the conventional organoruthenium compound of Chemical Formula 5. On the other hand, when the deposition temperature is set to 400°C and 500°C, a ruthenium thin film can be deposited (tests No. 7 and No. 8). However, the oxygen concentration in the thin film was high. This would be ascribable to ingress of oxygen atoms that form the organoruthenium compound as a raw material.

Second embodiment: Cis-diethyl-bis(N,N'-diisopropyl-1,4-diaza-1,3-butadiene)ruthenium (II) (each of substituents R₁, R₄, R₅ and R₈ is an isopropyl group, and each of substituents R₉ and R₁₀ is an ethyl group) was produced as an organoruthenium compound.

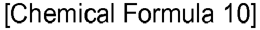

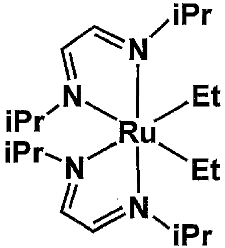

0.56 g (1.3 mmol) of cis-dichloro-bis(N,N'-diisopropyl-1,4-diaza-1,3-butadiene)ruthenium (II) was added in a flask containing 20 mL of tetrahydrofuran, and dissolved, and the solution was cooled with ice. 5 mL of an ethylmagnesium bromide/tetrahydrofuran solution (5 mmol; concentration: 1.0 M) was added to the ice-cooled solution. The mixed solution was stirred at room temperature for 1 hour to be reacted. The reaction formula here is as described below.

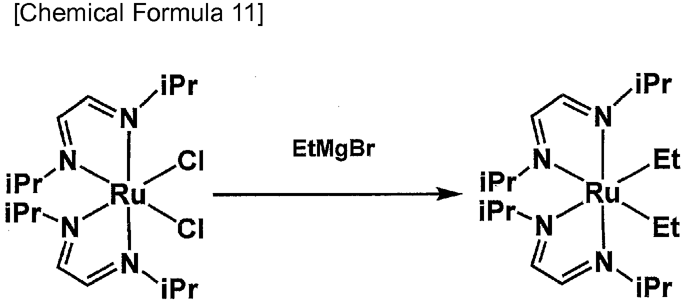

After completion of the reaction, the reaction product was concentrated to obtain a muddy reaction mixture. The resulting muddy reaction mixture was subjected to extraction operation with hexane, and the extract liquid was concentrated to obtain a reddish-violet solid. The solid was purified by a sublimation method to obtain 0.19 g (2.87 mmol) of an organoruthenium compound as a specified substance to be produced (yield: 43%). The organoruthenium compound was confirmed to be cis-diethyl-bis(N,N'-diisopropyl-1,4-diaza-1,3-butadiene)ruthenium (II) from ¹H-NMR analysis (¹H NMR(benzene-d₆): 8.05(2H, s), 7.80(2H, s), 5.11-5.04(2H, m), 3.86-3.80(2H, m), 2.10-2.01(2H, m), 1.43(6H, d), 1.41-1.36(8H, overlap), 1.17(6H, d), 0.94(6H, t), 0.41(6H, d)).

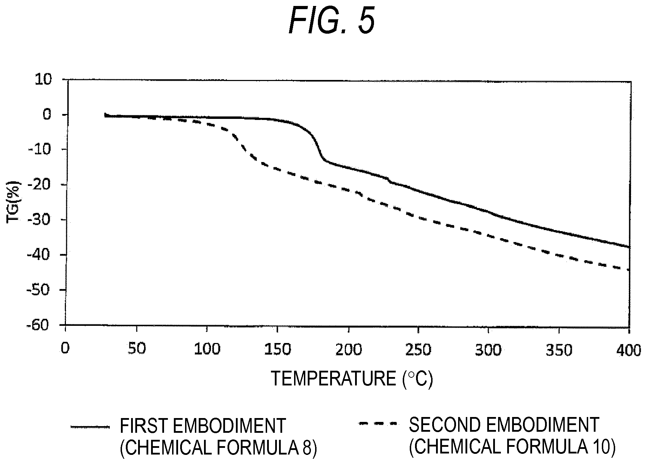

For the organoruthenium compound produced in the second embodiment, a change in weight was measured under an atmospheric pressure by TG-DTA analysis. Analysis conditions were the same as in the first embodiment. The results are shown in Fig. 5. In Fig. 5, the change in weight is also shown for comparison with the compound in the first embodiment. From Fig. 5, it can be confirmed that the organoruthenium compound in the second embodiment is smoothly decomposed in the air.

It is recognizable that cis-diethyl-bis(N,N'-diisopropyl-1,4-diaza-1,3-butadiene)ruthenium (II) as the organoruthenium compound in this embodiment is a compound capable of being decomposed at a lower temperature as compared to cis-dimethyl-bis(N,N'-diisopropyl-1,4-diaza-1,3-butadiene)ruthenium (II) as the organoruthenium compound produced in the first embodiment. The organoruthenium compound can be expected for use in deposition at a further low temperature.

### Industrial Applicability

The chemical vapor deposition raw material according to the present invention includes an organoruthenium compound having high thermal stability, has a moderate vapor pressure, and is excellent in deposition property at a low temperature. Ruthenium can be deposited even with hydrogen or the like as a reaction gas. The present inventive raw material is suitably used as a thin film electrode material of a semiconductor device such as DRAM or FERAM.

## Claims

1. A chemical vapor deposition raw material for producing a ruthenium thin film or a ruthenium compound thin film by a chemical deposition method, comprising an organoruthenium compound in which two diazadiene ligands and two alkyl ligands are coordinated to ruthenium, the organoruthenium being represented by the following formula: 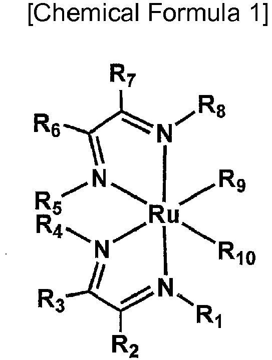 wherein each of substituents R₁ to R₈ of the diazadiene ligand is hydrogen, or a hydrocarbon group with a carbon number of 1 or more and 4 or less; two or more substituents selected from substituents R₁ to R₄ may be bonded together to form a cyclic structure with a carbon atom or a nitrogen atom to which the substituents are directly bonded; two or more substituents selected from substituents R₅ to R₈ may be bonded together to form a cyclic structure with a carbon atom or a nitrogen atom to which the substituents are directly bonded; and each of substituents R₉ and R₁₀ as alkyl ligands is an alkyl group with a carbon number of 1 or more and 3 or less.

2. The chemical vapor deposition raw material according to claim 1, wherein at least any of substituents R₁, R₄, R₅ and R₈ is an alkyl group, and at least any of the alkyl groups is a linear or branched alkyl group with a carbon number of 1 or more and 4 or less.

3. The chemical vapor deposition raw material according to claim 1 or 2, wherein at least any of substituents R₂, R₃, R₆ and R₇ is an alkyl group, and at least any of the alkyl groups is a methyl group.

4. A chemical deposition method of a ruthenium thin film or a ruthenium compound thin film, comprising preparing a raw material gas by vaporizing a raw material including an organoruthenium compound, and heating the raw material gas while introducing the raw material gas to a substrate surface,
Wherein the method uses the chemical vapor deposition raw material defined in any of claims 1 to 3 as the raw material.
